Europäisches Patentamt

European Patent Office

Office européen des brevets

(19)

(11) **EP 0 840 602 B1**

(12) **FASCICULE DE BREVET EUROPEEN**

(45) Date de publication et mention
de la délivrance du brevet:
**02.10.2002 Bulletin 2002/40**

(51) Int Cl.⁷: **A61K 31/11**

(21) Numéro de dépôt: **96924958.0**

(22) Date de dépôt: **09.07.1996**

(86) Numéro de dépôt international:
**PCT/FR96/01068**

(87) Numéro de publication internationale:
**WO 97/002814 (30.01.1997 Gazette 1997/06)**

(54) **UTILISATION D'UN RETINOIDE POUR LA PREPARATION D'UN MEDICAMENT ANTIBACTERIEN**

**VERWENDUNG EINER RETINOIDE ZUR HERSTELLUNG EINES ANTIBAKTERIELLEN MITTELS**

**USE OF A RETINOID FOR PREPARING AN ANTIBACTERIAL DRUG**

(84) Etats contractants désignés:
**AT BE CH DE DK ES FI FR GB GR IE IT LI LU MC
NL PT SE**

(30) Priorité: **10.07.1995 FR 9508300**

(43) Date de publication de la demande:
**13.05.1998 Bulletin 1998/20**

(73) Titulaire: **Pierre Fabre Dermo-Cosmetique
92100 Boulogne (FR)**

(72) Inventeurs:
• **SAURAT, Jean-Hilaire
CH-1206 Genève (CH)**
• **LAGARDE, Isabelle
F-31520 Ramonville-Saint-Agne (FR)**
• **GOORIS, Eric
F-31540 Pompertuzat (FR)**
• **COUSSE, Henri
F-31860 Pins Justaret (FR)**

(74) Mandataire: **Ahner, Francis et al
Cabinet Régimbeau
20, rue de Chazelles
75847 Paris cedex 17 (FR)**

(56) Documents cités:
**EP-A- 0 253 393          EP-A- 0 391 033
US-A- 4 034 114          US-A- 5 188 817**

• **BR. J. DERMATOL., vol. 126, no. 4, 1992, pages
362-366, XP002015197 R.E.A. WILLIAMS ET AL.:
"Staphylococcus aureus and intra-nasal
mupirocin in patients receiving isotretinoin for
acne."**
• **DERMATOL. CLIN. , vol. 11, no. 1, - 1993 pages
201-206, XP002015198 R.E.A. WILLIAMS ET AL.:
"the staphylococci: importance of their control
in the management of skin disease."**
• **J. CHEMOTHER., vol. 1, no. 6, - 1989 pages
374-376, XP002015199 A.C. FLEMETAKIS ET
AL.: "Effects of synthetic retinoids on the growth
of bacteria and their susceptibility to
antibiotics."**

**Description**

La présente invention concerne de nouvelles applications de composés appartenant au groupe des rétinoïdes.

Les rétinoïdes constituent un groupe de composés dérivés du rétinol, ou vitamine A.

Parmi les composés représentatifs de ce groupe on peut citer les esters de rétinol, l'acide rétinoïque, aussi appelé vitamine A acide, et le rétinal, sous forme de ses différents isomères.

Ces produits ont déjà été proposés dans diverses applications. La demande EP 253 393 concerne des méthodes de traitement par les rétinoïdes des dommages provoqués par l'exposition de la peau au soleil et à la lumière. La demande EP 391 033 revendique l'utilisation de dérivés du rétinal pour le traitement d'un ensemble de troubles tels que les rides, les verrues, les désordres de la kératinisation et le vieillissement de la peau. Le brevet US 4 034 114 avait déjà décrit la préparation de compositions topiques à base de rétinal pour le traitement des kératoses.

De manière inattendue, la Demanderesse a maintenant trouvé que les rétinoïdes possèdent des propriétés antibactériennes, utiles en particulier pour des applications en dermatologie et/ou cosmétologie.

Cette activité est particulièrement marquée sur les bactéries à Gram positif.

Certains Gram positifs font partie de la flore cutanée physiologique, mais certaines souches peuvent devenir pathogènes lors de modifications physiologiques (perturbation de la puberté) ou modification de l'environnement.

Le germe le plus pathogène avec complications possibles, est le staphylocoque doré (Staphylococcus aureus). C'est un germe à haut potentiel de multiplication parfois porteur de facteurs de résistances antibiotiques. Le portage de Staphylococcus aureus constitue un problème majeur. Il expose à des staphylococcies récidivantes, ainsi qu'à une transmission inter-humaine. 11 implique l'adhésion du germe à la peau, puis sa multiplication. Les antibiotiques disponibles n'agissent que sur la multiplication, par exemple les sels d'érythromycine, la clindamycine, l'acide fusidique.

La bactéricidie d'un produit se quantifie en termes de chute logarithmique de la population microbienne par rapport au temps. Or, il faut par exemple 48 heures à l'érythromycine pour faire chuter la population microbienne de 2 Log 10(1).

Les topiques antiseptiques peuvent également être utilisés, tels les sels d'ammonium quaternaires, les sels de chlorhexidine. Cependant ces produits présentent une activité cytotoxique intrinsèque, qui a pour conséquence de ralentir la cicatrisation cutanée.

Il existe donc bien un réel besoin pour un produit topique antistaphyloccique bactéricide c'est-à-dire à cinétique d'activité rapide sur le germe précité, qui posséderait, en plus, des propriétés trophiques pour la peau lésée.

C'est pourquoi la présente invention a pour objet l'utilisation d'un rétinoïde pour la préparation d'un médicament antibactérien, plus particulièrement d'un médicament bactéricide.

Cependant, les rétinoïdes selon l'invention peuvent également être utilisés pour préparer un médicament ayant une activité bactériostatique.

Selon un de ses aspects, l'invention concerne l'utilisation d'un rétinoïde comme décrit précédemment pour la préparation d'un médicament inhibant l'adhésion des bactéries aux cornéocytes.

Le médicament antibactérien selon l'invention se présentera de préférence sous forme d'une composition pharmaceutique comportant des excipients adaptés pour une application par voie topique.

Selon l'un des aspects de l'invention, la composition contient en outre un adjuvant de pénétration, par exemple le méthylal.

De préférence, les rétinoïdes sont choisis dans le groupe comprenant le rétinaldéhyde tout trans, le 9 cis-rétinaldéhyde et le 13 cis-rétinaldéhyde.

Les propriétés vitaminiques A et antibactériennes des compositions selon l'invention les rendent particulièrement adaptées pour corriger les lésions cutanées susceptibles de se surinfecter, les affections primitivement non microbiennes s'étant surinfectées, toutes lésions prurigineuses excoriées de l'adulte ou de l'enfant, les acnés surinfectés par un staphylocoque (doré ou epidermidis), certaines plaies microbiennes aux commissures des lèvres et du nez ; elles permettent aussi de traiter et de prévenir le portage de staphylocoques dorés dans les gites classiques.

Cette dernière indication résulte de l'association de l'effet bactéricide et de l'effet d'inhibition d'adhésions aux cornéocytes. Elle pourra notamment être mise en oeuvre dans le traitement des mains des infirmières.

En outre, la Demanderesse a mis en évidence pour le rétinal un effet d'augmentation de la synthèse du collagène, et de la prolifération épidermique, qui favorisent la cicatrisation et la régénération tissulaire. L'invention fournit donc un moyen pour préparer un médicament bactéricide d'action rapide, en particulier sur les bactéries Gram positif et qui accélère la réparation des petites lésions.

Ces propriétés nouvelles des rétinoïdes peuvent selon un autre aspect de l'invention être mises en oeuvre dans une méthode de traitement cosmétique pour corriger les rougeurs cutanées, caractérisée en ce qu'on applique localement un composé appartenant au groupe des rétinoïdes. Le rétinoïde est de préférence choisi dans le groupe comprenant : rétinaldéhyde tout trans, 9 cis-rétinaldéhyde et 13 cis-rétinaldéhyde.

Dans les compositions conformes à l'invention, la concentration en rétinoïdes, particulièrement en rétinaldéhyde, est de préférence située dans l'intervalle compris entre 0,01 et 0,5%, de préférence encore entre 0,01 et 0,05%.

Le rétinaldéhyde et ses isomères dont le 9 et le 13 cis-rétinaldéhyde possèdent une activité ciblée sur les Gram positifs, en particulier du genre Staphylococcus dont le staphylocoque doré. De plus, le rétinaldéhyde possède une activité inhibant d'adhésion des Gram positifs aux cornéocytes pour des doses infrabactériostatiques. L'efficacité a pu être mise en évidence par le calcul des concentrations minimales inhibitrices et bactéricides sur des souches polyrésistantes issues de prélèvements cutanés isolés en milieu hospitalier et par études de l'adhésion du staphylocoque aux cornéocytes selon Cole et Silverber (1986).

Le niveau d'efficacité, de l'ordre du microgramme, a été prouvé pour des souches de staphylocoques plurirésistantes notamment aux aminosides, tétracyclines et macrolides. L'efficacité bactéricide a pu être chiffrée. En effet, le rétinal à 0,1% a une D. value (temps nécessaire pour faire chuter la population du nombre de germes) sur Staphylococcus aureus 6538 P de 14,8 minutes avec un coefficient de corrélation de 0,999 en ce qui concerne la droite de régression linéaire reliant la population de germes au temps de contact germe-rétinaldéhyde.

Une utilisation de rétinoïdes selon l'invention comprend également toutes les étapes de formulation avec des excipients pharmaceutiquement et/ou cosmétologiquement acceptables connus de l'homme du métier tels que parfums, antioxydants, tensioactifs, conservateurs, stabilisants. On peut citer le BHT, BHA, ou les dérivés de tocophérol.

Les compositions peuvent notamment être présentés sous forme de crème, lotion, émulsion, etc.

Les exemples qui suivent sont destinés à illustrer l'invention sans aucunement en limiter la portée.

Exemple 1

Matériel et Méthode

1. Matériel

Les souches

    Staphylococcus aureus 6538 P
    Staphylococcus aureus de pathologies cutanées isolées de l'hôpital Rangueil Toulouse - (France)

Les milieux de culture

Détermination de la CMI et D. Value

  .  Bouillon Trypticase soja (Biomérieux)

Détermination de la CNB et D. Value

  .  Gélose Trypticase soja (Biomérieux)

L'appareillage

  .  Eau distillée stérile
  .  Microplaques stériles Nunc R Nunclon à 96 puits
  .  Tubes à essais stériles
  .  Ensemenceur manuel type Denley stérilisé extemporanément
  .  Micropipette Titerteck et cones stériles adaptés
  .  Pipette Ependorff et cones stériles adaptés
  .  Hotte à flux laminaire Bioblock Scientific
  .  Etuve à 32° C Jouan
  .  Spectropholomètre PRIM et cuves adaptées

. Bille de verre stériles

Les produits

Les solutions-mère des produits cités ci-dessous ont été préparées dans l'alcool puis dissoutes dans l'eau distillée stérile de façon à être en final à 200 µg/ml et environ 10° éthanolique (sous lumière jaune).

. Rétinal all Trans
. Rétinal 9 Cis (Sigma R 5754 - lot 62 H 84 43)
. Rétinal 13 Cis (Sigma R 6256 - lot 81 H 8440)

2. Méthode

Concentration Minimale Inhibitrice = CMI

Une distribution de 100 µl de bouillon Trypticase soja est réalisée dans chaque microcupule de la microplaque stérile (A).
Un témoin stérilité du milieu et un autre témoin de la vigueur de la souche sont réalisés en parallèle sur la microplaque.
L'ensemencement de la microplaque (A) a lieu sous la hotte après préparation de l'inoculum bactérien comme suit :
Les souches ayant été préalablement repiquées la veille sur gélose Trypticase soja, un inoculum $10^8$ germes/ml est dispersé dans l'eau distillée stérile puis agité au vortex.
Les billes de verre assurant une bonne dispersion des souches, la mesure de la densité permet de préparer un inoculum adéquat par rapport à un étalonnage pré-établi et validé.
La répartition de la souche a lieu dans une deuxième microplaque (B) stérile par distribution de 100 µl d'inoculum par microcupule.
L'ensemencement a lieu par un repiquage de la microplaque (B) vers la microplaque (A), il se réalise ainsi une dilution au 1/100 de l'inoculum : la microplaque A contenant $10^6$ germes par ml, peut être mise à l'étuve 24 h à 32° C.
La lecture de la plus grande dilution donnant lieu à une clarté identique au témoin stérilité du milieu, constitue la concentration minimale inhibitrice.

Concentration Minimale Bactéricide = CMB

Le repiquage de la microplaque A sur une gélose trypticase soja préalablement coulée, permet après culture de 24-48 h à 32° C, la lecture de la concentration CMB = plus petite concentration ne permettant pas de croissance sur la gélose.

|  | St. aureus | St. epidermis |
|---|---|---|
| RETINALDEHYDE ALL TRANS | 0,0003 % | 0,0001% |
|  | 0,0002 % | 0,0003 % |
| RETINAL 9 Cis | 0,0003 % | 0,0001 % |
|  | 0,0005 % | 0,0006 % |
| RETINAL 13 Cis | 0,0006 % | 0,0001 % |
|  | 0,0005 % | 0,0006 % |

Ces résultats ont été confirmés sur 11 souches issues de pathologies cutanées et les moyennes des CMI et CMB sont :

$$CMI = 2,26 + 0,77 \ \mu g/ml$$

$$CMB = 11,93 + 6,77 \ \mu g/ml$$

La D. value du Rétinal à 0,1 % P/V dans une solution éthanolique à 20 % V/V a été recherchée sur St. aureus 6538 P.

La cinétique des prélèvements microbiens a été réalisée aux temps 15', 30', 60', 4h et 6h. L'inoculum de départ étant de $1,0.10^4$ germes par ml, la neutralisation se réalise d'après les temps cités dans du Bouillon Trypticase Soja, supplémenté par 10 % de Tween 80. Le neutralisant a été validé au préalable.

Les résultats suivants sont obtenus :

| Temps | 15 | 30 | 60 | 4h | 6h | D. value |
|---|---|---|---|---|---|---|
| Rétinal 0,1% | $1,4.10^3$ | $1,3.10^2$ | 0 | 0 | 0 | 14,8 mn r = 0,99 |
| Log | 3,15 | 2,11 | | | | |

Comme la molécule rétinal est difficile à stabiliser, le minimum d'excipients sera retenu.

Ainsi, les excipients à propriétés antimicrobiennes complémentaires du spectre, seront préférés.

Les isomères 9 Cis et 13 Cis présentant un même niveau d'efficacité, une bonne rémanence microbienne cutanée, permettra une seule application par jour.

Dans le cas de lésions, la molécule possédant des propriétés kératoplastiques, un effet cicatrisant complètera l'efficacité curative de la préparation.

Exemple 2

| Rétinaldéhyde | 0,01 à 0,05 g |
|---|---|
| Alcool à 30° QSP | 100 |

Exemple 3

Afin d'améliorer la pénétration cutanée, un adjuvant pourra être retenu.

| Rétinaldéhyde | 0,01 à 0,05 g |
|---|---|
| Méthylal qsp | 100 |

Exemple 4

| Rétinaldéhyde | 0,01 à 0,05 g |
|---|---|
| Propylène glycol ou | |
| Butylène glycol | 15 à 20 g |
| Eau distillée qsp | 100 |

Les formules citées dans les exemples ne sont pas exhaustives et pourront être complétées par les concentrations de 1% de BHT ou de 0,02 de BHA ou de dérivé de tocophérol.

REFERENCES

1. Thabaut A. et Meyran M. (1989)
Activité bactéricide comparée de quatorze antibiotiques sur staphylococcus aureus.
Path. Biol. 37 : 321 - 328.

2. Cole G.W. ; Silvergerg N.L (1986)
The adherence of staphylococcus aureus to human corneocytes.

Arch. Dermatol. 122 : 166 - 169.

**Revendications**

1.  Utilisation d'un rétinoïde pour la préparation d'un médicament antibactérien.

2.  Utilisation selon la revendication 1 pour la préparation d'un médicament bactéricide.

3.  Utilisation d'un rétinoïde selon l'une des revendications 1 ou 2 pour la préparation d'un médicament inhibant l'adhésion des bactéries aux cornéocytes.

4.  Utilisation selon l'une des revendications 1 à 3, **caractérisée en ce que** le rétinoïde est choisi dans le groupe comprenant le rétinaldéhyde tout trans, le 9 cis-rétinaldéhyde et le 13-cis rétinaldéhyde.

5.  Utilisation selon l'une des revendications 1 à 4, **caractérisée en ce qu'**il s'agit d'un médicament antibactérien actif par voie topique.

6.  Utilisation d'un rétinoïde selon l'une des revendications 1 à 5, **caractérisée en ce que** le médicament est actif sur les bactéries gram positif, en particulier du genre Staphylococcus.

**Patentansprüche**

1.  Verwendung eines Retinoids für die Herstellung eines antibakteriellen Medikaments,

2.  Verwendung nach Anspruch 1 für die Herstellung eines bakteriziden Medikaments.

3.  Verwendung eines Retinoids gemäß einem der Ansprüche 1 oder 2 für die Herstellung eines Medikaments, das die Anhaftung von Bakterien an Corneocyten hemmt.

4.  Verwendung nach einem der Ansprüche 1 bis 3, **dadurch gekennzeichnet, dass** das Retinoid ausgewählt ist aus der Gruppe bestehend aus all-trans-Retinaldehyd, 9-cis-Retinaldehyd und 13-cis-Retinaldehyd.

5.  Verwendung nach einem der Ansprüche 1 bis 4, **dadurch gekennzeichnet, dass** es sich um ein antibakterielles Medikament handelt, das auf topischem Weg wirksam ist.

6.  Verwendung eines Retinoids nach einem der Ansprüche 1 bis 5, **dadurch gekennzeichnet, dass** das Medikament bei grampositiven Bakterien, insbesondere der Gattung Staphylococcus, wirksam ist.

**Claims**

1.  Use of a retinoid, for preparing an antibacterial drug.

2.  Use according to Claim 1, for preparing a bactericidal drug.

3.  Use of a retinoid according to one of Claims 1 and 2, for preparing a drug which inhibits the adhesion of bacteria to corneocytes.

4.  Use according to one of Claims 1 to 3, **characterized in that** the retinoid is chosen from the group comprising all-trans retinaldehyde, 9-cis-retinaldehyde and 13-cis-retinaldehyde.

5.  Use according to one of Claims 1 to 4, **characterized in that** it involves an antibacterial drug which is active topically.

6.  Use of a retinoid according to one of Claims 1 to 5, **characterized in that** the drug is active on Gram-positive bacteria, in particular of the genus Staphylococcus.